# EUROPEAN PATENT SPECIFICATION

(11) **EP 2 650 000 B1**
(45) Date of publication and mention of the grant of the patent: **15.03.2017**
(21) Application number: 13001555.5
(22) Date of filing: 26.03.2013
(51) Int. Cl.: A61K 31/555, A61P 35/00

(54) **Utilization of copper complexes involving 2-phenyl-3-hydroxy-4(1H)-quinolinone and 1,10-phenanthroline derivatives for the preparation of drugs for the treatment of tumour diseases**
Verwendung von Kupferkomplexen mit 2-Phenyl-3-hydroxy-4(1H)-quinolinon und 1,10-Phenanthrolinderivaten zur Herstellung von Arzneimitteln zur Behandlung von Tumorerkrankungen
Utilisation de complexes de cuivre impliquant des dérivés de 2-phényl-3-hydroxy-4(1H)-quinolinone et 1,10-phénanthroline pour la préparation de médicaments pour le traitement des maladies tumorales

(30) Priority: 10.04.2012 CZ 20120242
(43) Date of publication of application: 16.10.2013
(73) Proprietor: Univerzita Palackého, 771 47 Olomouc (CZ)
(72) Inventor: Trávnícek, Zdenek, Stepánov u Olomouce, 78313 (CZ); Vanco, Ján, Herspice, 68401 (CZ); Buchtík, Roman, Olomouc, 77900 (CZ); Dvorák, Zdenek, Olomouc, 77900 (CZ)
(74) Representative: Soukup, Petr

(56) References cited:
- CZ-B6- 303 009
- ROMAN BUCHTÍK ET AL: "Synthesis, characterization, DNA interaction and cleavage, and in vitro cytotoxicity of copper(ii) mixed-ligand complexes with 2-phenyl-3-hydroxy-4(1H)-quinolinone", DALTON TRANSACTIONS, vol. 40, no. 37, 2011, page 9404, XP55065972, ISSN: 1477-9226, DOI: 10.1039/c1dt10674k
- SERMENT-GUERRERO J ET AL: "Genotoxicity of the copper antineoplastic coordination complexes casiopeinas", TOXICOLOGY IN VITRO, ELSEVIER SCIENCE, GB, vol. 25, no. 7, 6 May 2011 (2011-05-06), pages 1376-1384, XP028313480, ISSN: 0887-2333, DOI: 10.1016/J.TIV.2011.05.008 [retrieved on 2011-05-13]
- ZDENEK TRÁVNÍCEK ET AL: "Cellular responses induced by Cu(II) quinolinonato complexes in human tumor and hepatic cells", CHEMISTRY CENTRAL JOURNAL, vol. 6, no. 1, 20 December 2012 (2012-12-20), page 160, XP55066347, ISSN: 1752-153X, DOI: 10.1007/s00216-009-3180-3
- Hiroshi Nishiwaki ET AL: "Experimental Studies on the Antitumor Effect of Ethidium Bromide and Related Substances1", CANCER RESEARCH, 1 October 1974 (1974-10-01), pages 2699-2703, XP55199386, Retrieved from the Internet: URL:http://cancerres.aacrjournals.org/cont ent/34/10/2699.full.pdf [retrieved on 2015-07-01]
- CREAVEN B S ET AL: "Anticancer and antifungal activity of copper(II) complexes of quinolin-2(1H)-one-derived Schiff bases", INORGANICA CHIMICA ACTA, ELSEVIER BV, NL, vol. 363, no. 14, 25 November 2010 (2010-11-25), pages 4048-4058, XP027422677, ISSN: 0020-1693, DOI: 10.1016/J.ICA.2010.08.009 [retrieved on 2010-08-10]
- CHEW HEE NG ET AL: "Selective anticancer copper(ii)-mixed ligand complexes: targeting of ROS and proteasomes", METALLOMICS, vol. 6, no. 4, 1 January 2014 (2014-01-01) , page 892, XP055255190, GB ISSN: 1756-5901, DOI: 10.1039/c3mt00276d
- Isabel Gracia-Mora ET AL: "Knigth's Move in the Periodic Table, From Copper to Platinum, Novel Antitumor Mixed Chelate Copper Compounds, Casiopeinas, Evaluated by an in Vitro Human and Murine Cancer Cell Line Panel", METAL-BASED DRUGS, vol. 8, no. 1, 1 January 2001 (2001-01-01) , pages 19-28, XP055255214, IL ISSN: 0793-0291, DOI: 10.1155/MBD.2001.19

## Description

### Field of the Invention

This invention relates to the copper complexes involving 2-phenyl-3-hydroxy-4(1 H)-quinolinone and derivatives of 1,10-phenanthroline for utilization in the preparation of drugs for the treatment of tumour diseases, specifically for the treatment of human lung adenocarcinoma, human cervix epitheloid carcinoma, human malignant melanoma, human ovarian carcinoma, human *cisplatin*-resistant ovarian carcinoma and human prostate carcinoma.

### Background of the Invention

Copper complexes constitute an important group of compounds in the research of antitumour active substances, because they represent a possible alternative to the currently used coordination compounds of platinum, among which e.g. *cisplatin* (*cis-*diammine-dichlorido platinum(II) complex), see structural formula A, as the most world-wide used representative of such type of compounds still belongs.

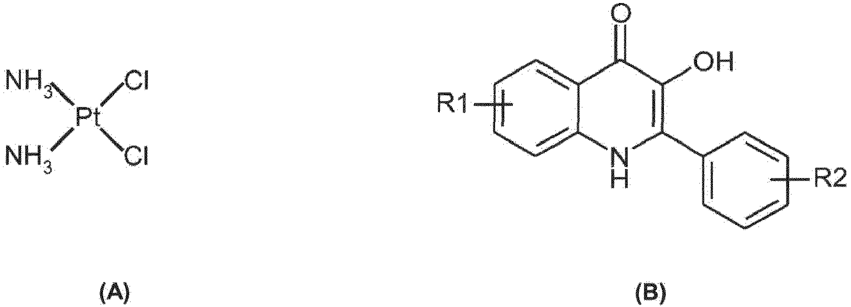

The derivatives of 2-phenyl-3-hydroxy-4(1H)-quinolinone of the general formula (B) exhibit a wide spectrum of biological activities themselves, which depend on the substitution on the heterocyclic aromatic ring (R1) as well as on the substitution (R2) on the phenyl group in the position 2 (Hradil, P. et al., J. Heterocyclic Chem., 2004, 3 (41), 375-379, Soural, M. et al., Eur. J. Med. Chem., 2006, 41, 467-474, Soural, M. et al., J. Comb. Chem., 2007, 9, 793-796, patent applications WO2008028427, CZ 20100476). In the given references above, the preparation and cytotoxic effects of 2-phenyl-3-hydroxy-4(1 H)-quinolinone derivatives are described. These organic compounds represent aza-analogues of naturally occurring flavones, whose biological activities are described in a number of patents, e.g. EP 0558245, US 5238954, US 5733920, WO 2009129372, EP 0803503. In connection with the fact that 2-phenyl-3-hydroxy-4(1H)-quinolinone derivatives have not been widely described in the literature to date, it is quite straightforward that their coordination compounds have been studied even less. The first described copper(II) complexes of these derivatives are as follows: bis(4-oxo-2-phenyl-1,4-dihydroquinolin-3-olato-κO⁴)copper(II), [Cu(qui)₂], and {2-(benzoylamino)benzoato-κO}-(4-oxo-2-phenyl-1,4-dihydroquinolin-3-olato-κO⁴)-(N1,N1',N2,N2'-tetramethyl-1,2-ethandiamine-κN1,-κN2)copper(II), [Cu(babe)(qui)(tmen)], and their crystal-solvates with *N,N'-*dimethylformamide (Czaun, M. et al., Chem. Commun., 2004, 8, 1004-1005, Kaizer, J. et al., Monograph series, 20th ICCBC, Smolenice, Slovak Republic, June 5-10, 2005, 111-121).

It has to be also mentioned that copper(II) complexes containing 1,10-phenanthroline and its derivatives exhibit a wide spectrum of biological activities as well. The well-known group of such compounds is called Casiopeinas®, which are Cu(II) complexes of 1,10-phenanthrolines or 2,2'-bipyridines with aminoacids (Serment-Guerrero, J. et al., Toxicology in Vitro 2011, 25(7), 1376-1384.; Bravo-Gomez M. E. et al., J. Inorg. Biochem. 2009, 103, 299-309).

The characterization and selected biological activities of the claimed complexes are described in the publications of Buchtik R. et al., Dalton Trans., 2011, 40, 9404-9412, Buchtik R. et al., J. Inorg. Biochem., 2012, 116, 163-171 and Trávníček Z. et al., Chem. Centr. J., 2012, 6, 160. In the granted national patent CZ 303009 B6, *in vitro* biological activity of the claimed complexes against the human osteosarcoma (HOS) and breast carcinoma (MCF7) cell lines is mentioned. The activity of the claimed complexes is compared with the commercially used cytostatic *cisplatin,* and in some cases, it reached more than tenfold efficiency in comparison to *cisplatin.*

### Summary of the invention

The invention provides copper complexes in the oxidation state +II and their crystal-solvates having the general formula [Cu(qui)(L2)]X, and expressed by the structural formula (I), 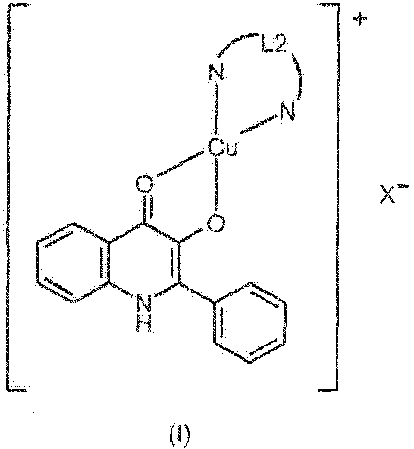 where the symbol qui stands for the 2-phenyl-3-hydroxy-4(1H)-quinolinonate(1-) anion, and the symbol L2 stands for a differently substituted 1,10-phenanthroline (phen) derivative, independently chosen from the group of: 1,10-phenanthroline, 4-methyl-1,10-phenanthroline, 5-methyl-1,10-phenanthroline, 5-nitro-1,10-phenathroline, 5-chloro-1,10-phenanthroline, 5,6-dimethyl-1,10-phenanthroline, 4,7-diphenyl-1,10-phenanthroline, and 3,4,7,8-tetramethyl-1,10-phenanthroline, expressed by the specific formulas as [Cu(qui)(phen)]X, [Cu(qui)(4-methylphen)]X, [Cu(qui)(5-methylphen)]X, [Cu(qui)(5-nitrophen)]X, [Cu(qui)(5-chlorophen)]X, [Cu(qui) (5,6-dimethylphen)]X, [Cu(qui)(4,7-diphenylphen)]X, [Cu(qui)(3,4,7,8-tetramethylphen)]X, and the symbol X stands for a monoanion independently chosen from the group of: F⁻, Cl⁻, Br, I⁻, NO₃⁻, BF₄⁻, BPh₄⁻, HCOO⁻, CH₃COO⁻, ClO₄⁻, HSO₄⁻; for ? use? the treatment of human lung carcinoma and/or human cervix epitheloid carcinoma and/or human malignant melanoma and/or human ovarian carcinoma and/or human *cisplatin-*resistant ovarian carcinoma and/or human prostate carcinoma.

In the course of ongoing research, it has been unexpectedly found out that the copper complexes involving 2-phenyl-3-hydroxy-4(1H)-quinolinone, of the structural motif (**I**) and expressed by the general formula [Cu(qui)(L2)]X and their crystal-solvates, exhibit not only significant *in vitro* anticancer activity against the cell lines of human osteosarcoma (HOS) and human breast carcinoma (MCF7), but also a much broader anticancer effect. Concretely, a significant *in vitro* anticancer activity against the cell lines of human lung adenocarcinoma (A549), human cervix epitheloid carcinoma (HeLa), human malignant melanoma (G361), human ovarian carcinoma (A2780), human *cisplatin-*resistant ovarian carcinoma (A2780R) and human prostate carcinoma (LNCaP) has been found. These findings, which have not been included either in the utility model UV 2010-23634 or patent CZ 303009, significantly increase the applicability of the claimed compounds in the treatment of malignant neoplasms. It has to be emphasized that the resulting copper(II) complex itself, and not the reaction components, has to be considered as a biological activity bearer, because the reactants themselves have been found to be completely inactive in terms of cytotoxicity against the tested cancer cell lines.

A huge advantage of the submitted invention is the finding that the claimed complexes are generally more active, e.g. by more than two orders of magnitude against the human *cisplatin*-resistant ovarian carcinoma cell line (A2780R), as compared to *cisplatin.*

The wide range of anticancer effects and therefore the spectrum of possible utilizations of the claimed complexes, together with the strong ability to overcome some of mechanisms of the resistance towards the *cisplatin*-resistant ovarian carcinoma cell line (A2780R), represent the valuable contribution of the submitted invention to the current state of the art.

### Description of the figures

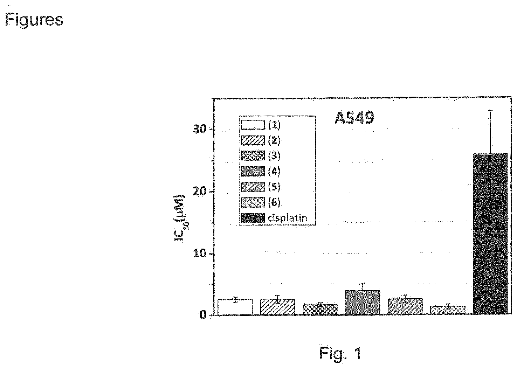

The concrete examples of the invention are documented by the attached drawings, where
- Fig. 1 represents a graph of the *in vitro* antitumour activity of the specific complexes against human lung adenocarcinoma cell line (A549) and comparison of their activities with *cisplatin:* (2-phenyl-4-oxo-1,4-dihydroquinolin-3-olato-κ²O³:O⁴)(1,10-phenanthroline-κ²N¹:N¹⁰)copper(II) nitrate monohydrate [Cu(qui)(phen)]NO₃·H₂O (**1**); (2-phenyl-4-oxo-1,4-dihydroquinolin-3-olato-κ²O³:O⁴)(5-methyl-1,10-phenanthroline-κ²N¹:N¹⁰)copper(II) nitrate monohydrate [Cu(qui)(mphen)]NO₃·H₂O (**2**), (2-phenyl-4-oxo-1,4-dihydroquinolin-3-olato-κ²O³:O⁴)(4,7-diphenyl-1,10-phenanthroline-κ²N¹:N¹⁰)copper(II) nitrate monohydrate [Cu(qui) (bphen)]NO₃·H₂O (**3**), (2-phenyl-4-oxo-1,4-dihydroquinolin-3-olato-κ²O³:O⁴) (1,10-phenanthroline-κ²N¹:N¹⁰)copper(II) tetrafluoroborate [Cu(qui)(phen)]BF₄ (**4**), (2-phenyl-4-oxo-1,4-dihydroquinolin-3-olato-κ²O³:O⁴)(5-methyl-1,10-phenanthroline-κ²N¹:N¹⁰)copper(II) tetrafluoroborate monohydrate [Cu(qui) (mphen)]BF₄·H₂O (**5**) and (2-phenyl-4-oxo-1,4-dihydroquinolin-3-olato-κ²O³:O⁴) (4,7-diphenyl-1,10-phenanthroline-κ²N¹:N¹⁰) copper(II) tetrafluoroborate [Cu(qui)(bphen)]BF₄ (**6**), where Hqui = 2-phenyl-3-hydroxy-4(1H)-quinolinone, where phen = 1,10-phenanthroline, mphen = 5-methyl-1,10-phenanthroline and bphen = bathophenanthroline.
- Fig. 2 represents a graph of the *in vitro* antitumour activity of complexes **1**-**6** against human malignant melanoma cancer cell line (G361) and comparison of their activities with *cisplatin.*
- Fig. 3 represents a graph of the *in vitro* antitumour activity of complexes **1**-**6** against human cervix epitheloid carcinoma cell line (HeLa) and comparison of their activities with *cisplatin.*
- Fig. 4 represents a graph of the *in vitro* antitumour activity of complexes **1**-**6** against human ovarian carcinoma cell line (A2780) and comparison of their activities with *cisplatin*.
- Fig. 5 represents a graph of the *in vitro* antitumour activity of complexes **1**-**6** against human *cisplatin*-resistant ovarian carcinoma cell line (A2780R) and comparison of their activities with *cisplatin.*
- Fig. 6 represents a graph of the *in vitro* antitumour activity of complexes **1**-**6** against human prostate carcinoma cell line (LNCaP) and comparison of their activities with *cisplatin*.

### Examples

In the following section, the invention is documented but not limited, by the concrete examples of its realization. The invention range is unambiguously limited by the invention claims.

The *in vitro* anticancer activity of the prepared complexes listed as the examples below was determined by the MTT assay. The method is based on the ability of metabolic-active cells to reduce (by mitochondrial dehydrogenases) the yellow tetrazolium dye (3-(4,5-dimethylthiazol-2-yl)-2,5-diphenyltetrazolium bromide (MTT)) to the blue formazane end-product. Because the reduction proceeds only in the living cells, the cytotoxicity of various compounds can be determined using this process. The water-insoluble formazane is quantified after dissolving in dimethyl sulfoxide (DMSO) with the solution of ammonia on a spectrophotometer (ELISA reader). The *in vitro* anticancer activity testing was performed on the following human cancer cell lines: lung adenocarcinoma (A549), cervix epitheloid carcinoma (HeLa), malignant melanoma (G361), ovarian carcinoma (A2780), *cisplatin*-resistant ovarian carcinoma (A2780R) and prostate carcinoma (LNCaP). The cell lines were maintained in plastic vessels in the DMEM medium (5 g/l glucose, 2 mM glutamine, 100 U/mL penicillin, 100 µg/mL streptomycin, 10% foetal calf serum and sodium bicarbonate) for cell cultures. The 80 µL of cell suspensions (ca. 1.25 × 10⁵ cells per mL) were pipetted onto 96-well plastic microtitration plates. These were further pre-incubated at 37 °C in the CO₂ atmosphere for 24 hours. The tested copper complexes and *cisplatin,* used as positive and therapeutic standard, were dissolved in *N,N'*-dimetylformamide in the concentrations up to 50 mM which served as stock solutions. For the experiment, the stock solutions were diluted 1000 x in the incubation medium (maximum concentration of 50.0 µM). After removal of the cultivation media, the mixtures of appropriate concentration were added to the cancer cells for the incubation period of 24 hours. The MTT analysis was performed spectrophotometrically (TECAN, Schoeller Instruments LLC) at 540 nm.
The concentrations of the tested complexes (µM) toxic for 50% of cancer cells (inhibition concentration IC₅₀) were calculated from the dose-response curves.

### Example 1: In vitro anticancer activity of the tested complexes against human lung adenocarcinoma cell line (A549) and comparison of their activities with cisplatin.

The determination of *in vitro* anticancer activity of the studied compounds **1**-**6** and *cisplatin* against human lung adenocarcinoma cell line (A549) was performed by the MTT assay.

The inhibition concentrations of the studied complexes IC₅₀ (µM) are equal to 2.5±0.4 (complex **1**), 2.5±0.6 (complex **2**), 1.7±0.3 (complex **3**), 3.9±1.2 (complex **4**), 2.5±0.6 (complex **5**) and 1.3±0.4 (complex **6**), and 25.8±7.1 for *cisplatin* (see Fig. 1).

### Example 2: In vitro anticancer activity of the tested complexes against human malignant melanoma cancer cell line (G361) and comparison of their activities with cisplatin.

The determination of *in vitro* anticancer activity of the studied compounds **1**-**6** and *cisplatin* against human malignant melanoma cancer cell line (G361) was performed by the MTT assay.

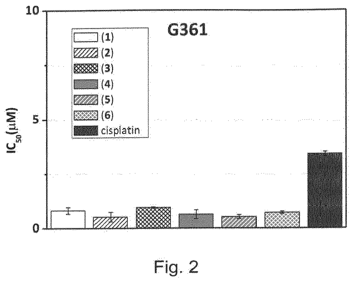

The inhibition concentrations of the studied complexes IC₅₀ (µM) are equal to 0.82±0.15 (complex **1**), 0.53±0.22 (complex **2**), 0.966±0.03 (complex **3**), 0.64±0.20 (complex **4**), 0.53±0.09 (complex **5**) and 0.72±0.06 (complex **6**), and 3.4±0.1 for *cisplatin* (see Fig. 2).

### Example 3: In vitro anticancer activity of the tested complexes against human cervix epitheloid carcinoma cell line (HeLa) and comparison of their activities with cisplatin.

The determination of *in vitro* anticancer activity of the studied compounds **1**-**6** and *cisplatin* against human cervix epitheloid carcinoma cell line (HeLa) was performed by the MTT assay.

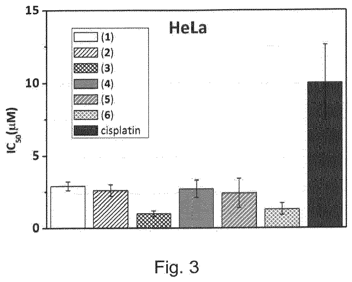

The inhibition concentrations of the studied complexes IC₅₀ (µM) are equal to 2.9±0.3 (complex **1**), 2.6±0.4 (complex **2**), 1.0±0.2 (complex **3**), 2.7±0.6 (complex **4**), 2.4±1.0 (complex **5**) and 1.3±0.4 (complex **6**), and 10.0±2.6 for *cisplatin* (see Fig. 3).

### Example 4: In vitro anticancer activity of the tested complexes against human ovarian carcinoma (A2780) cell line and comparison of their activities with cisplatin.

The determination of *in vitro* anticancer activity of the studied compounds **1**-**6** and *cisplatin* against human ovarian carcinoma cell line (A2780) was performed by the MTT assay.

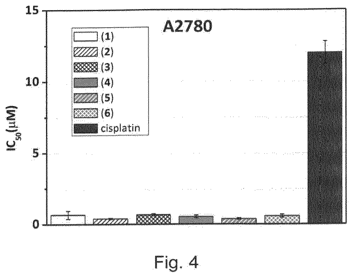

The inhibition concentrations of the studied complexes IC₅₀ (µM) are equal to 0.65±0.28 (complex **1**), 0.38±0.03 (complex **2**), 0.66±0.07 (complex **3**), 0.52±0.10 (complex **4**), 0.36±0.05 (complex **5**) and 0.57±0.11 (complex **6**), and 12.0±0.8 for *cisplatin* (see Fig. 4).

### Example 5: In vitro anticancer activity of the tested complexes against human cisplatin-resistant ovarian carcinoma cell line (A2780R) and comparison of their activities with cisplatin.

The determination of *in vitro* anticancer activity of the studied compounds **1-6** and *cisplatin* against human *cisplatin*-resistant ovarian carcinoma cell line (A2780R) was performed by the MTT assay.

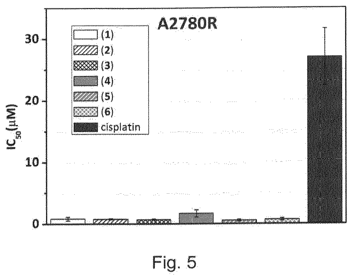

The inhibition concentrations of the studied complexes IC₅₀ (µM) are equal to 0.83±0.30 (complex **1**), 0.78±0.07 (complex **2**), 0.73±0.09 (complex **3**), 1.7±0.6 (complex **4**), 0.56±0.15 (complex **5**) and 0.70±0.20 (complex **6**), and 27.0±4.6 for *cisplatin* (see Fig. 5).

### Example 6: In vitro anticancer activity of the tested complexes against human prostate carcinoma cell line (LNCaP) and comparison of their activities with cisplatin.

The determination of *in vitro* anticancer activity of the studied compounds **1-6** and *cisplatin* against human prostate carcinoma cell line (LNCaP) was performed by the MTT assay.

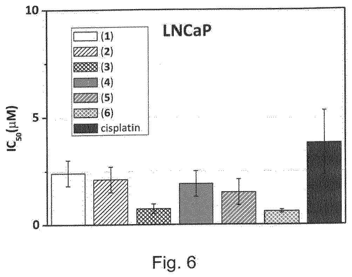

The inhibition concentrations of the studied complexes IC₅₀ (µm) are equal to 2.4±0.6 (complex **1**), 2.1±0.6 (complex **2**), 0.74±0.21 (complex **3**), 1.9±0.6 (complex **4**), 1.5±0.6 (complex **5**) and 0.62±0.08 (complex **6**), and 3.8±1.5 for *cisplatin* (see Fig. 6).

## Claims

1. Copper complexes in the oxidation state +II and their crystal-solvates having the general formula [Cu(qui)(L2)]X, and expressed by the structural formula (I), 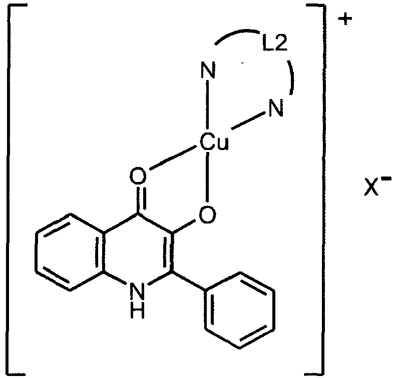 where the symbol qui stands for the 2-phenyl-3-hydroxy-4(1H)-quinolinonate(1-) anion, and the symbol L2 stands for a differently substituted 1,10-phenanthroline (phen) derivative, independently chosen from the group: 1,10-phenanthroline, 4-methyl-1,10-phenanthroline, 5-methyl-1,10-phenanthroline, 5-nitro-1,10-phenanthroline, 5-chloro-1,10-phenanthroline, 5,6-dimethyl-1,10-phenanthroline, 4,7-diphenyl-1,10-phenanthroline, and 3,4,7,8-tetramethyl-1,10-phenanthroline, expressed by the specific formulas as [Cu(qui)(phen)]X, [Cu(qui)(4-methylphen)]X, [Cu(qui)(5-methylphen)]X, [Cu(qui)(5-nitrophen)]X, [Cu(qui)(5-chlorophen)]X, [Cu(qui) (5,6-dimethylphen)]X, [Cu(qui)(4,7-diphenylphen)]X, [Cu(qui)(3,4,7,8-tetramethylphen)]X, and the symbol X stands for a monoanion independently chosen from the group of: F⁻, Cl⁻, Br⁻, I⁻, NO₃⁻, BF₄⁻, BPh₄⁻, HCOO⁻, CH₃COO⁻, ClO₄⁻, HSO₄⁻; for use in the treatment of human lung carcinoma and/or human cervix epithelioid carcinoma and/or human malignant melanoma and/or human ovarian carcinoma and/or human *cisplatin*-resistant ovarian carcinoma and/or human prostate carcinoma.

## Patentansprüche

1. Kupferkomplexe mit der Oxidationszahl + II und ihre Kristallsolvate mit der allgemeinen Formel [Cu(qui)(L2)]X, und der Strukturformel (I) 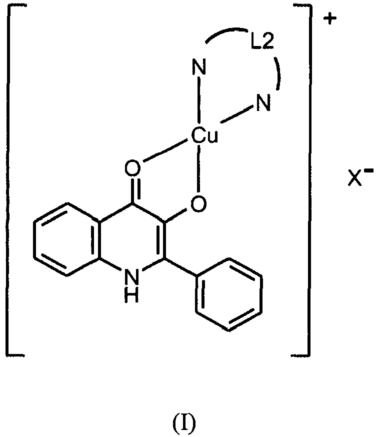 wobei qui für das 2-Phenyl-3-hydroxy-4(1H)-chinolinat(1-) Anion und L2 für ein anders substituiertes 1,10-phenanthrolin (phen) Derivat steht, das unabhängig aus der Gruppe 1,10-phenanthrolin, 4-methyl-1,10-phenanthrolin, 5-methyl-1,10-phenanthrolin, 5-nitro-1,10-phenanthrolin, 5-chloro-1,10-phenanthrolin, 5,6-dimethyl-1,10-phenanthrolin, 4,7-diphenyl-1, 10-phenanthrolin und 3,4,7,8-tetramethyl-1,10-phenanthrolin gewählt wird, dargestellt durch die spezifischen Formeln [Cu(qui)(phen)]X, [Cu(qui)(4-methylphen)]X, [Cu(qui)(5-methylphen)]X, [Cu(qui)(5-nitrophen)]X, [Cu(qui)(5-chlorophen)]X, [Cu(qui)(5,6-dimethylphen)]X, [Cu(qui)(4,7-diphenylphen)]X, [Cu(qui)(3,4,7,8-tetramethylphen)]X, wobei X für unabhängig aus der Gruppe der Monoanionen F⁻, Cl⁻, Br⁻, I⁻, NO₃⁻, BF₄⁻, BPh₄⁻, HCOO⁻, CH₃COO⁻, ClO₄⁻, HSO₄⁻ gewählt wird für die Verwendung zur Behandlung von humanen Lungenkarzinomen und/oder humanen Zervixepithelkarzinomen und/oder humanen malignen Melanomen und/oder humanen Ovarkarzinomen und/oder humanen *cisplatin*-resistenten Ovarkarzinomen und/oder humanen Prostatakarzinomen.

## Revendications

1. Complexes de cuivre au degré d'oxydation +11 et ses formes solvatées et non-solvatées de cristaux ayant la formule générale [Cu(qui)(L2)]X, et représentées par la formule structurale (I), 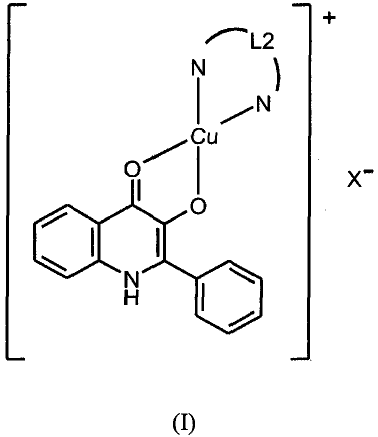 où le symbole « qui » signifie l'anion 2-phényl-3-hydroxy-quinoléinate(1-) et le symbole L2 signifie un dérivé différemment substitué de 1,10-phénantroline, choisi indépendamment du groupe: 1,10-phénantroline, 4-méthyl-1,10-phénantroline, 5-méthyl-1,10-phénantroline, 5-nitro-1,10-phénantroline, 5-chloro-1,10-phénantroline, 5,6-diméthyl-1,10-phénantroline, 4,7-diphényl-1,10-phénantroline et 3,4,7,8-tétramethyl-1,10-phénantroline, exprimés par la formule spécifique : [Cu(qui)(4-méthylphén)]X, [Cu(qui)(5-méthylphén)]X, [Cu(qui)(4-nitrophén)]X, [Cu(qui)(5-chlorophén)]X, [Cu(qui)(5,6-méthylphén)]X, [Cu(qui)(4,7-dipheylphén)]X, [Cu(qui)(3,4,7,8-tétraméthylphén)]X et le symbole X signifie un monoanion choisi indépendamment du groupe : F⁻, Cl⁻, Br⁻, I⁻, NO₃⁻, BF₄⁻, BPh₄⁻, HCOO⁻, CH₃COO⁻, ClO₄⁻, HSO₄⁻; pour utiliser en traitement de carcinome pulmonaire humain et/ou carcinome épithélioïde humain du col de l'utérus et/ou mélanome malin humain et/ou carcinome humain de l'ovaire et/ou carcinome humain de l'ovaire résistant au cisplatine et/ou carcinome humain de la prostate.
